# EUROPEAN PATENT APPLICATION

(11) **EP 1 020 193 A1**
(43) Date of publication of application: **19.07.2000**
(21) Application number: 98944298.3
(22) Date of filing: 29.09.1998
(51) Int. Cl.: A61K 47/10

(54) **ORAL PREPARATION**

(30) Priority: 30.09.1997 JP 26544197
(71) Applicant: DAIICHI PHARMACEUTICAL CO., LTD., Chuo-ku, Tokyo 103-0027 (JP)
(72) Inventor: II, Noritaka, Daiichi Pharm. Co., Ltd Tokyo, Toyka 134-0081 (JP); MURAKAMI, Toshio, Daiichi Pharm. Co., LtdTokyo, Tokyo 134-0081 (JP)
(74) Representative: Kinzebach, Werner, Dr.
(86) International application number: JP9804374
(87) International publication number: WO9916470

(57) **Abstract**

An oral administration preparation having excellent taking ability, in which unpleasant tastes of drugs are improved by jointly adding a sugar alcohol having a heat of dissolution of -20 cal/g or less and a pH adjusting agent to the drugs having unpleasant tastes.

## Description

### TECHNICAL FIELD:

This invention relates to an oral administration preparation in which unpleasant tastes, particularly bitterness, of drugs are improved.

### BACKGROUND ART:

In general, taking of oral administration preparations of drugs having unpleasant tastes (a bitter taste, a sharp taste, a puckery taste and the like) causes problems such as a difficulty in swallowing then because of the unpleasant tastes of the drugs themselves. Thus, in order to mask unpleasant tastes of drugs at the time of their taking, they are made into pharmaceutical preparations as capsules, sugar coated tablets, film coated tablets, three layer tablets, syrups and the like dosage forms.

Also, regarding granules, powders and fine subtilaes of drugs having unpleasant tastes, certain measures such as 1) a method in which film coating is carried out and 2) a method in which drugs are dispersed in melted waxes which are subsequently solidified and pulverized are used to mask the unpleasant tastes of drugs. However, the pharmaceutical preparations produced by the method 1) are not disintegrated in the oral cavity, so that they cause problems in that they feel rough to the tongue and cause pain by getting in between artificial teeth. The preparations produced by the method 2) also have a problems of reduced bioavailability due to inferior dissolution ability of the drugs in digestive tracts.

In addition to the above methods, several methods are known in which unpleasant tastes, particularly bitterness, of drugs are improved by additive agents.

For example, JP-A-2-76826 (the term "JP-A" as used herein means an "unexamined published Japanese patent application") describes that bitterness of an acidic drug (acid addition salt of a basic drug) becomes undetectable when menthol and an alkaline substance are added thereto, and JP-A-4-327529 describes an oral administration preparation having improved bitterness, in which a nucleus containing acid addition salt of a basic drug is coated with a weakly alkaline compound. Also, JP-A-6-206824 describes a drug composition having reduced bitterness, in which an alkaline earth metal oxide and an alkaline earth metal hydroxide are added to a drug having bitterness, and JP-A-6-157312 describes a preparation of bitterness-improved terfenadine dry syrup granules, in which xylitol is added. In addition, JP-A-8-99904 describes an H2 blocker solid preparation having improved bitterness and taking ability, in which a sugar alcohol having a heat of dissolution of -60 KJ/kg or less is added.

When bitterness is improved by additive agents, their usually used examples include saccharides such as sucrose, glucose and fructose, sugar alcohols such as erythritol, D-mannitol, D-sorbitol, xylitol, maltitol, maltose, lactitol and hydrogenated maltose starch syrup and sweeteners such as saccharin, aspartame, glycyrrhizic acid, stevia and thaumatin.

However, When bitterness is improved by the addition of the aforementioned saccharides, sugar alcohols or the like, it poses a problem in that saccharides or sugar alcohols must be formulated in large amounts. That is, it is necessary to use at least 25 parts by weight or more of saccharides or sugar alcohols based on 1 part by weight of a drug having bitterness, and it is necessary to use 50 parts by weight or more or 100 parts by weight or more of than when the bitterness is further improved. Since dosage forms of this case are syrups, troches, drops (sweets) and the like, there is a disadvantage in that the pharmaceutical preparations must be made into relatively large forms. Particularly, in the case of a drug whose dose is 100 mg or more per once, the method to add saccharides or sugar alcohols is effective in reducing bitterness but, for improving the bitterness to a completely undetectable level, it has limitations in terms of the size and amount of the pharmaceutical preparation which can be taken, so that it is practically difficult to prepare tablets, granules, powders, fine subtilaes and the like.

In addition, though the addition of an alkaline substance is effective in reducing bitterness, but the effect to reduce bitterness has a limitation even by the formulation of a large amount of the alkaline substance, so that it is difficult to improve the bitterness to a completely undetectable level by the alkaline substance alone.

In recent years, attempts have been made to develop tablets, granules and the like dosage forms which dissolve or disintegrate quickly in the oral cavity, so that even children and the aged can take than easily without water. However, since many drugs have bitter tastes, improvement of the bitterness is a great problem in developing such pharmaceutical preparations, and sufficiently satisfactory preparations have not been obtained yet.

The object of the invention is to provide oral administration preparations having excellent taking ability, in which unpleasant tastes of drugs are improved to a completely undetectable level by the addition of a small amount of an additive agent.

### DISCLOSURE OF THE INVENTION:

As a result of intensive investigation, the present inventors have found that unpleasant tastes, particularly bitter tastes, can be improved to a completely undetectable level by the joint addition of a sugar alcohol having a heat of dissolution of -20 cal/g or less and a pH adjusting agent to a drug having an unpleasant taste, and thereby accomplished the invention.

Accordingly, the invention relates to an oral administration preparation which contains a drug having an unpleasant taste, a sugar alcohol having a heat of dissolution of -20 cal/g or less and a pH adjusting agent.

### BEST MODE FOR CARRYING OUT THE INVENTION:

The invention provides an oral administration preparation which is small in size and can be easily taken, because the amount of a sugar alcohol to be added to improve unpleasant tastes of drugs is reduced by the joint use of a pH adjusting agent. Illustratively, it provides an oral administration preparation having more smaller amount of dose as a pharmaceutical preparation, in which the amount of a sugar alcohol to be added is reduced to at least 1/5, preferably 1/10, more preferably 1/20, in comparison with the case of its single use, by the joint use of a pH adjusting agent.

Though the drug having an unpleasant taste according to the invention is not particularly limited, examples of the compound having an unpleasant taste, especially a bitter taste, include a compound which has at least one basic group in its structure, an acid addition salt of the compound, a solvate of the compound, a solvate of an addition salt of the compound and the like. In this case, the basic group means a primary amino group, a secondary amino group, a tertiary amino group, a quaternary amino group or the like group, and its illustrative examples include amino group, amidino group, methylamino group and the like.

The method for reducing unpleasant tastes according to the invention can be divided into three types [a] to c)] depending on the kind of drugs. That is,
a) when the drug is a compound which has a basic group in its structure, its solubility in the oral cavity is reduced by inhibiting dissociation of the base to keep its un-dissociated form (molecular type), by increasing pH in the oral cavity to the pKa value or more of the drug by the use of a pH adjusting agent. In addition, taste of the drug is changed (to a taste of oil and fat) by increasing its solubility in fat and, furthermore, the unpleasant taste of the drug is reduced.
b) When the drug is an amphoteric compound which has a basic group and an acidic group in its structure, its taste is changed by increasing pH in the oral cavity to the pKa value or more of the acidic group (e.g., carboxyl group) in the structure by the use of a pH adjusting agent, thereby effecting dissociation of the group and formation of an intramolecular salt or a salt with the pH adjusting agent. In addition, dissociation of the basic group is inhibited by increasing pH in the oral cavity to the pKa value or more of the group and, furthermore, the unpleasant taste of the drug is reduced.
c) When the drug is an acid addition salt of a compound which has a basic group or an acid addition salt of an amphoteric compound, because compounds are made into acid addition salts in many cases in order to increase solubility of these drugs in water, solubility of the drug in the oral cavity is reduced by converting it into free form through elimination of the acid addition salt making use of a pH adjusting agent and, furthermore, the unpleasant taste of the drug is reduced.

The following can be exemplified as the drugs having unpleasant tastes, especially bitter tastes. Examples of the compound which has a basic group in its structure include cimetidine, famotidine, nizatidine, acetaminophen, epirizole, pyrazinamide, caffeine, ethionamide, carvedilol, aminophylline, sulpyrine, theophylline, diphenhydramine, metoclopramide, phenylbutazone, phenobarbital, chloramphenicol and the like.

Examples of the amphoteric compound which has a basic group and an acidic group in its structure include those which have the aforementioned basic group and carboxyl group, sulfonic group, phosphoric group or the like acidic group in its structure, such as tranexamic acid, epsilon-aminocaproic acid, gamma-aminobutyric acid, nalidixic acid, levofloxacin, ofloxacin, L-tryptophan, L-leucine, L-isoleucine, ampicillin, enoxacin and the like.

Examples of the acid addition salt of a compound having a basic group include salts of a compound having a basic group with hydrochloric acid, nitric acid, sulfuric acid and the like mineral acids and salts of a compound having a basic group with acetic acid, tartaric acid, maleic acid, citric acid and the like organic acids, such as ticlopidine hydrochloride, ranitidine hydrochloride, roxatidine acetate HCl, imipramine hydrochloride, ephedrine hydrochloride, chlorpromazine hydrochloride, diphenhydramine hydrochloride, tetracycline hydrochloride, doxycycline hydrochloride, naphazoline hydrochloride, noscapine hydrochloride, papaverine hydrochloride, hydralazine hydrochloride, dextromethorphan hydrobromide, timepidium bromide, chlorpheniramine maleate, alimamazine tartarate, pilsicainide hydrochloride, N-methylscopolamine methylsulfate, clopidogrel sulfate, cinepazide maleate and the like.

Examples of the acid addition salt of an amphoteric compound include salts of an amphoteric compound with hydrochloric acid, nitric acid, sulfuric acid and the like mineral acids and salts of an amphoteric compound with acetic acid, tartaric acid, maleic acid, citric acid and the like organic acids, such as cetraxate hydrochloride, arginine hydrochloride, histidine hydrochloride, lysine hydrochloride, lysine acetate and the like. In addition, according to the invention, crude drugs which contain the basic group-containing compounds or amphoteric compounds as their components and extracted products (extracts, tinctures and the like) of the crude drugs are also included in the drugs of the invention having unpleasant tastes. Examples of such crude drugs include corydalis tuber, phellodendri cortex, coptidis rhizoma, strychni semen, ephedrae harba, ipecac, scopoliae rhizoma, belladonna leaf, sophorae radix and the like.

According to the invention, the improvement of unpleasant tastes of drugs is carried out by adding a pH adjusting agent, but the pH adjusting agent having a too high pH value will cause a stimulus by the pH adjusting agent itself in the oral cavity when the oral administration preparation of the invention is taken. Because of this, according to the invention, the pKa value of a drug having an unpleasant taste or the pH value of 1% (w/v) aqueous solution or 1% (w/v) aqueous suspension of the drug having an unpleasant taste is from 2 to 11, preferably from 3 to 10, more preferably from 4 to 9.

According to the invention, cimetidine, famotidine, nizatidine, ranitidine hydrochloride and the like H₂ blockers and tranexamic acid, ticlopidine hydrochloride, clopidogrel sulfate, cetraxate hydrochloride and the like can be exemplified as the drugs having unpleasant tastes, especially bitter tastes, which are suited for applying to the invention.

The pH adjusting agent of the invention is not particularly limited, with the proviso that it can make a compound having a basic group, as a drug which shows an unpleasant taste in the oral cavity, into its un-dissociated form (molecular type) by inhibiting dissociation of the basic group, or can make an addition salt of the compound having a basic group or an addition salt of an amphoteric compound into its free form. Illustratively, a preferred pH adjusting agent may show, in its 1% (w/v) aqueous solution or 1% (w/v) aqueous suspension form, a pH value of equal to or higher than the pKa value of a drug as a compound having a basic group or an amphoteric compound having a basic group and an acidic group or a pH value of equal to or higher than the pH value of a 1% (w/v) aqueous solution or 1% (w/v) aqueous suspension of a drug as an acid addition salt of the compound having a basic group or an acid addition salt of the amphoteric compound having a basic group and an acidic group, more preferably a value larger than the just described pKa value or pH value of the drug. Particularly, it is desirable that the aforementioned pH value of the pH adjusting agent is larger than the aforementioned pKa value or pH value of the drug, by a factor of preferably from 0.5 to 7, wore preferably from 1 to 3. Regarding pH value of the pH adjusting agent of the invention, it is preferably from 3 to 12, more preferably from 4 to 11, most preferably from 5 to 10. In this case, the term "1% (w/v) aqueous solution" means that 1 g of a solute is dissolved in 100 ml of a solvent. The case of the suspension is the same as the aqueous solution.

Examples of the pH adjusting agent include alkali metal salts of organic acids, alkaline earth metal salts of organic acids, amino acids, metal salts of amino acids and weakly acidic to weakly alkaline (illustratively pH 5 to 10) inorganic compounds.

Illustratively, salts of citric, malic acid, tartaric acid, succinic acid, maleic acid, fumaric acid, malonic acid, acetic acid, lactic acid and the like organic acids with sodium, potassium and the like alkali metals can be exemplified as the alkali metal salts of organic acids, and salts of the just described organic acids with magnesium, calcium and the like alkaline earth metals can be exemplified as the alkaline earth metal salts of organic acids.

Regarding the amino acids, glycine, alanine, leucine, isoleucine, valine, serine, threonine, aspartic acid, glutamine, asparagine, glutamine, lysine, arginine, histidine and the like can be exemplified, and salts of the just described amino acids with sodium, potassium and the like alkali metals can be exemplified as the alkali metal salts of amino acids.

In addition, examples of the weakly acidic to weakly alkaline inorganic compounds include dried aluminum hydroxide gel, magnesium aluminosilicate, magnesium silicate, synthetic aluminum silicate, synthetic hydrotalcite, magnesium oxide, aluminum magnesium hydroxide, aluminum hydroxide gel, aluminum hydroxide-sodium bicarbonate co-precipitate, aluminum hydroxide-magnesium carbonate dried mixed gel, aluminum hydroxide-magnesium carbonate-calcium carbonate co-precipitate, magnesium hydroxide, sodium bicarbonate, magnesium carbonate, precipitated calcium carbonate, magnesium aluminometasilicate, anhydrous dibasic calcium phosphate, dibasic calcium phosphate, cuttlefish bone, shijueming, ostreae testa, dibasic potassium phosphate, monobasic potassium phosphate, dibasic sodium phosphate, disodium hydrogen phosphate anhydrous, monobasic sodium phosphate, tribasic sodium phosphate, potassium carbonate, potassium bicarbonate, sodium carbonate, sodium polyphosphate and the like.

According to the invention, alkali metal salts of organic acids and weakly acidic to weakly alkaline inorganic compounds are preferred as the pH adjusting agent, of which dried aluminium hydroxide, magnesium aluminosilicate, magnesium silicate, synthetic aluminum silicate, synthetic hydrotalcite , magnesium oxide, aluminum magnesium hydroxide, sodium citrate, sodium malate, sodium tartarate, sodium bicarbonate, disodium hydrogen phosphate anhydrous and precipitated calcium carbonate are more preferred. Most preferred among them are sodium citrate, sodium slate, sodium tartarate, sodium bicarbonate, disodium hydrogen phosphate anhydrous and precipitated calcium carbonate.

The pH adjusting agent of the invention is added only for the purpose of adjusting the pH value in the oral cavity to a level equal to or higher than the pKa value of a drug or the pH value of a 1% (w/v) aqueous solution or 1% (w/v) aqueous suspension of the drug, so that it is not necessary to add it in a large amount. The pH adjusting agent may be added in an amount of from 0.1 to 200 parts by weight, preferably from 0.2 to 50 parts by weight, more preferably from 0.3 to 10 parts by weight, most preferably from 0.5 to 7 parts by weight, based on 1 part by weight of a drug having an unpleasant taste.

Since the pH adjusting agent is not added in a large amount, pH value in the oral cavity is controlled and the solubility of a drug having an unpleasant taste is thereby reduced in the oral cavity, but, because solubility of the drug is pH -dependently reversible, the drug is neutralized by gastric acid once transferred into the stomach and its original solubility is recovered. In consequence, effect of the pH adjusting agent on the absorption of the drug is hardly generated.

Cimetidine, which is one of the drugs having unpleasant tastes (bitterness) to which the invention can suitably be applied, has a pKa value of 7.1. Its solubility in water is 30 mg/ml (25°C) at pH 6.5 but is reduced to 5.3 mg/ml (25°C) at pH 8.3. Also, cetraxate hydrochloride is an acid addition salt of an amphoteric compound, and its pKa values are 4.5 (carboxyl group) and 10.5 (amino group). Its solubility in water is 27 mg/ml (22°C) at pH 2.8 but is reduced to 4.4 mg/ml (22°C) at pH 3.3 and to 0.4 mg/ml (22°C) at pH 5.9. On the basis of such information, solubility of a drug in the oral cavity can be controlled by a pH adjusting agent, and furthermore, unpleasant taste (bitterness) of the drug can be reduced. In addition, unpleasant taste of the drug can be improved to a completely undetectable level by the addition of a sugar alcohol having a heat of dissolution of -20 cal/g or less.

According to the invention, the sugar alcohol having a heat of dissolution of -20 cal/g or less means a case in which absorbed heat of dissolution is 20 cal/g or larger than that when a sugar alcohol is dissolved in water. Examples of such a sugar alcohol include erythritol (heat of dissolution: -42.9 cal/g), xylitol (heat of dissolution: -35 cal/g), mannitol (heat of dissolution: -28.9 cal/g), sorbitol (heat of dissolution: -24.1 cal/g) and the like, and erythritol is particularly preferable among these sugar alcohols, because larger absorbed heat of dissolution results in larger effect to improve unpleasant taste and smaller amount to be added.

When a sugar alcohol does not have a heat of dissolution of -20 cal/g or less, its effect to improve unpleasant taste is small, so that good taking feeling cannot be obtained unless considerably increasing its adding amount. For example, proper effect to improve unpleasant taste (bitterness) was not obtained when a sugar alcohol having a heat of dissolution of not -20 cal/g or less, namely maltitol (heat of dissolution: -5.5 cal/g), was added even in an amount of 20 parts by weight based on 1 part by weight of a drug having an unpleasant taste (bitterness) (see Test Example 3 which will be described later). Also, in the case of sucrose (heat of dissolution: -4.5 cal/g), glucose (heat of dissolution: -13.8 cal/g) and the like saccharides, their effect to improve unpleasant taste (bitterness) of a drug was small despite of their high sweetness, so that proper taking feeling was not obtained (see Test Example 3 which will be described later).

However, when a sugar alcohol having a heat of dissolution of -20 cal/g or less is used, unpleasant tastes of drugs are improved and an oral administration preparation having a refreshing feeling and good taking feeling on be obtained.

According to the invention, the sugar alcohol having a heat of dissolution of -20 cal/g or less may be added in an amount of from 0.1 to 50 parts by weight, preferably from 1 to 25 parts by weight, more preferably from 5 to 10 parts by weight, based on 1 part by weight of a drug having an unpleasant taste. Also, it may be 30% by weight or more, preferably from 30 to 90% by weight, more preferably from 40 to 70% by weight, based on the total weight of the pharmaceutical preparation.

Though there is no particular limitation regarding particle size of the sugar alcohol having a heat of dissolution of -20 cal/g or less according to the invention, it is desirable that it has a particle size of 500 µm or less in view of rough feeling in the mouth and the like points when it is used in an oral solid preparation.

Though not particularly limited, examples of the dosage form of the oral administration preparation of the invention include tablets, granules, powders, fine subtilaes, solutions, syrups and the like. In this connection, the tablets include chewable tablets, troches, drops and moldings which are quickly dissolved and disintegrated in the oral cavity and can therefore be taken without water, and they also include effervescent tablets which are dissolved when used. The granules, powders and fine subtilaes include dry syrups which are dissolved when used and granular preparations which are quickly dissolved and disintegrated in the oral cavity and can therefore be taken without water.

The oral administration preparation of the invention may contain generally used various pharmaceutical additives in such amounts that they do not spoil effects of the invention. Examples of such pharmaceutical additives include excipients, disintegrators, binders, lubricants, coloring agents, flavors, sweeteners, correctives and the like.

Examples of the excipients include lactose, sucrose, starch, microcrystalline cellulose, light anhydrous silicic acid, calcium silicate and the like. Examples of the disintegrators include low substituted hydroxypropylcellulose, carmellose, crospovidone, carmellose calcium, croscarmellose sodium and the like. Examples of the binders include hydroxypropylcellulose, hydroxypropylmethylcellulose, polyvinyl alcohol, polyvinylpyrrolidone and the like. Examples of the lubricants include magnesium stearate, calcium stearate, talc, sucrose esters of fatty acid and the like. Examples of the coloring agents include Food Yellow No. 5, Food Red No. 2, Food Blue No. 2, food lake dyes, yellow ferric oxide, titanium oxide and the like. Examples of the flavors include orange, lemon and the like various aromatics.

Examples of the sweeteners include aspartame, stevia, thaumatin, saccharin sodium, dipotassium glycyrrhizinate and the like. Aspartame is particularly preferable among these sweeteners, because it has an effect to remove salty taste generated by the addition of a sodium salt as a pH adjusting agent. Aspartame is added in an amount of from 0.01 to 2% by weight, preferably from 0.05 to 1% by weight, more preferably from 0.1 to 0.5% by weight, based on the total weight of the pharmaceutical preparation. Examples of the correctives include Ic-menthol, camphor, mentha, monosodium L-glutamate monohydrate, dibasic sodium inosinate, magnesium chloride and the like. Among then, L-menthol is particularly desirable, because it exerts a refreshing feeling and further increases the bitterness-improving effect. L-Menthol is added in an amount of from 0.01 to 2% by weight, preferably from 0.05 to 1% by weight, more preferably from 0.1 to 0.5% by weight, based on the total weight of the preparation.

These pharmaceutical additives may be added optionally at a proper step in producing the oral administration preparation.

The oral administration preparation of the invention can be produced by known methods for the production of oral administration preparations. For example, fluidized bed granulation, agitation granulation, rolling fluidized bed granulation, extrusion granulation, spray granulation, pulverization granulation and the like can be used as the granulation method of solid pharmaceutical preparations. The following illustratively describes a production method making use of fluidized bed granulation.

A drug having an unpleasant taste is mixed with a sugar alcohol having a heat of dissolution of -20 cal/g or less, a pH adjusting agent and, as occasion demands, lactose, corn starch and the like excipients, the mixture is made into granules by a fluidized bed granulation dryer using an aqueous solution of hydroxypropylcellulose, polyvinyl alcohol or the like binder, and then the granules, after adding aspartame if desired, are mixed using a mixer and made into powders, granules or fine subtilaes. Alternatively, the thus obtained granulation product may be mixed with a necessary amount of magnesium stearate, talc or the like lubricant and then treated with a tablet making machine in the usual way to obtain tablets or chewable tablets.

Also, as occasion demands, the drug having an unpleasant taste and the pH adjusting agent may be made into separate granular preparations in preparing the granules, which are then mixed with each other (multiple granulation method).

Preferred embodiments of the invention are as follows.
1. An oral administration preparation which contains a drug having an unpleasant taste, a sugar alcohol having a heat of dissolution of -20 cal/g or less and a pH adjusting agent.
2. The oral administration preparation according to the aforamentioned embodiment 1, wherein the drug having an unpleasant taste has a basic group in its structure.
3. The oral administration preparation according to the embodiment 1 or 2, wherein the drug having an unpleasant taste is a drug which has a bitter taste.
4. The oral administration preparation according to any one of the embodiments 1 to 3, wherein the drug having an unpleasant taste is an H₂ blocker.
5. The oral administration preparation according to the embodiment 4, wherein the H₂ blocker is a mixture of one or more compounds selected from the group consisting of cimetidine, famotidine, nizatidine and ranitidine hydrochloride.
6. The oral administration preparation according to any one of the embodiments 1 to 3, wherein the drug having an unpleasant taste is a mixture of one or more compounds selected from the group consisting of cimetidine, tranexamic acid and cetraxate hydrochloride.
7. The oral administration preparation according to any one of the embodiments 1 to 6, wherein the sugar alcohol having a heat of dissolution of -20 cal/g or less is a mixture of one or more compounds selected from the group consisting of erythritol, xylitol, mannitol and sorbitol.
8. The oral administration preparation according to any one of the embodiments 1 to 6, wherein the sugar alcohol having a heat of dissolution of -20 cal/g or less is erythritol.
9. The oral administration preparation according to any one of the embodiments 1 to 8, wherein the sugar alcohol having a heat of dissolution of -20 cal/g or less is from 0.1 to 50 parts by weight based on 1 part by weight of the drug having an unpleasant taste.
10. The oral administration preparation according to any one of the embodiments 1 to 8, wherein the sugar alcohol having a heat of dissolution of -20 cal/g or less is from 5 to 10 parts by weight based on 1 part by weight of the drug having an unpleasant taste.
11. The oral administration preparation according to any one of the embodiments 1 to 10, wherein pH value of a 1% (w/v) aqueous solution or 1% (w/v) aqueous suspension of the pH adjusting agent is equal to or higher than the pKa value of the drug having an unpleasant taste or equal to or higher than the pH value of a 1% (w/v) aqueous solution or 1% (w/v) aqueous suspension of the drug.
12. The oral administration preparation according to any one of the embodiments 1 to 11, wherein the pH adjusting agent is a mixture of one or more compounds selected from the group consisting of sodium bicarbonate, sodium dihydrogen phosphate anhydrous and precipitated calcium carbonate.
13. The oral administration preparation according to any one of the embodiments 1 to 12, wherein the pH adjusting agent is from 0.1 to 200 parts by weight based on 1 part by weight of the drug having an unpleasant taste.
14. The oral administration preparation according to any one of the embodiments 1 to 12, wherein the pH adjusting agent is from 0.5 to 7 parts by weight based on 1 part by weight of the drug having an unpleasant taste.
15. An oral administration preparation which contains from 5 to 10 parts by weight of a sugar alcohol having a heat of dissolution of -20 cal/g or less and from 0.5 to 7 parts by weight of a pH adjusting agent, based on 1 part by weight of an H₂ blocker.
16. The oral administration preparation according to any one of the embodiments 1 to 15, wherein it further contains a sweetener and/or a corrective agent.
17. The oral administration preparation according to any one of the embodiments 1 to 15, wherein it further contains aspartame and/or L-menthol.
18. The oral administration preparation according to any one of the embodiments 1 to 17, wherein the dosage forms are tablets, granules, powders, fine subtilaes, solutions or syrups.
19. A method for improving taking ability of an oral administration preparation containing a drug having an unpleasant taste, which is effected by including a sugar alcohol having a heat of dissolution of -20 cal/g or less and a pH adjusting agent.
20. The method for improving taking ability of an oral administration preparation according to the embodiment 19, wherein a sweetener and/or a corrective agent is further included.

The following describes the invention further in detail with reference to inventive and test examples, though the invention is not limited to these examples.

### [Inventive Example 1]

A 50 g portion of cimetidine (pKa: 7.1), 250 g of erythritol (manufactured by Nikken Chemicals: passed through a screen having an sieve opening of 350 µm), 225 g of precipitated calcium carbonate, 75 g of sodium bicarbonate, 33.5 g of corn starch and 6.5 g of aspartame were weighed, put into a fluidized bed granulation dryer, mixed for 3 minutes and then subjected to granulation under a spraying pressure of 1.5 kg/cm² and at a spraying solution rate of 15 ml/minute using 200 ml of a 5% (w/v) aqueous solution of hydroxypropylcellulose. After drying, the thus prepared granules were passed through a screen having an sieve opening of 1,000 µm to obtain a powder preparation (contains 100 mg of cimetidine in 1.3 g of the powder).

### [Inventive Example 2]

A 50 g portion of cimetidine (pKa: 7.1), 350 g of exythritol (manufactured by Nikken Chemicals: passed through a screen having an sieve opening of 350 µm), 100 g of precipitated calcium carbonate, 75 g of sodium bicarbonate, 32.1 g of corn starch, 30 g of microcrystalline cellulose and 6.5 g of aspartame were weighed, put into a fluidized bed granulation dryer, mixed for 3 minutes and then subjected to granulation under a spraying pressure of 1.5 kg/cm² and at a spraying solution rate of 15 ml/minute using 100 ml of a 2.5% (w/v) aqueous solution of hydroxypropylcellulose. After drying, the thus obtained granules were passed through a screen having an sieve opening of 1,000 µm and mixed with 0.6% by weight of magnesium stearate. Thereafter, the mixture was applied to a single punch tabletting machine and made into tablets, each tablet weighing 1,300 mg, with a ring punch of 18 mm in outer diameter and 6 mm in bore diameter, thereby obtaining chewable tablets (contains 100 mg of cimetidine in one tablet).

### [Test Example 1] Sensory test of bitterness (1)

A sensory test of bitterness was carried out using the cimetidine-containing solid preparations obtained in Inventive Example 1 and Inventive Example 2. The sensory test was carried out by a panel of five members, by keeping each preparation in the oral cavity for about 20 seconds and then judging the degree of bitterness based on the following evaluation criteria. The results are shown in Table 1.
- A:: Feel no bitterness.
- B:: Feel almost no bitterness.
- C:: Feel bitterness slightly.
- D:: Feel bitterness.
- E:: Feel bitterness strongly.

**Table 1**

| Panelist | 1 | 2 | 3 | 4 | 5 |
|---|---|---|---|---|---|
| Inventive Example 1 | A | A | A | A | A |
| Inventive Example 2 | A | A | A | A | A |

As is evident from the results shown in Table 1, all, of the five panelists felt no bitterness regarding the powder of Inventive Example 1 and tablets of Inventive Example 2.

### [Test Example 2] Sensory test of bitterness (2)

Powders of cimetidine, erythritol, sodium bicarbonate, precipitated calcium carbonate and aspartame were weighed at the respective weight ratios shown in Table 2 and mixed using a mortar, and a sensory test of bitterness was carried out using the thus obtained mixed powders. The sensory test was carried out by a panel of two members, by keeping each preparation in the oral cavity for about 20 seconds and then judging the degree of bitterness based on the following evaluation criteria. The results are shown in Table 2.
- A:: Feel no bitterness.
- A*:: Feel no bitterness but feel a salty taste.
- B:: Feel almost no bitterness.
- C:: Feel bitterness slightly.
- C*:: Feel bitterness slightly and also feel a salty taste.
- D:: Feel bitterness.
- E:: Feel bitterness strongly.

**Table 2**

| Composition No. | a | b | c | d | e | f | g | h | i | j | k |
|---|---|---|---|---|---|---|---|---|---|---|---|
| [Component] | | | | | | | | | | | |
| Cimetidine | 1 | 1 | 1 | 1 | 1 | 1 | 1 | 1 | 1 | 1 | 1 |
| Erythritol | - | 25 | 50 | 100 | - | 4 | 5 | 6 | 7 | 7 | 10 |
| Sodium bicarbonate | - | - | - | - | 2 | 1.5 | 1.5 | 1.5 | 2 | 2 | 2 |
| Precipitated | - | - | - | - | - | 4.5 | 4.5 | 4.5 | - | - | - |
| calcium carbonate | | | | | | | | | | | |
| Aspartame | - | - | - | - | - | - | - | - | - | 0.01 | 0.01 |

| (Sensory test result) | | | | | | | | | | | |
|---|---|---|---|---|---|---|---|---|---|---|---|
| Panelist A | E | C | A | A | C* | B | A | A | A* | A | A |
| Panelist B | E | C | B | A | C* | D | A | A | A* | A | A |

As is evident from the results shown in Table 2, in order to improve the bitter taste of cimetidine by the addition of only a sugar alcohol erythritol, 50 parts by weight or more, or 100 parts by weight for further improving the bitterness, of erythritol is required based on 1 part by weight of cimetidine (see compositions c and d). On the other hand, when the sugar alcohol is used jointly with a pH adjusting agent, the bitterness of cimetidine can be improved by adding 4 parts by weight or more, or 5 parts by weight or more for further improving the bitterness, of erythritol based on 1 part by weight of cimetidine, and also adding, as the pH adjusting agent, 2 parts by weight of sodium bicarbonate or 1.5 parts by weight of sodium bicarbonate and 4.5 parts by weight of precipitated calcium carbonate (see compositions f to i). In this connection, a salty taste was detected when 2 parts by weight of sodium bicarbonate was added, but it was found that the addition of 0.01 part by weight of aspartame was particularly desirable because of its effect to rerove the salty taste (see compositions j and k).

### [Test Example 3] Sensory test of bitterness (3)

Powders of cimetidine, xylitol, D-mannitol, D-sorbitol, maltitol, glucose, sucrose, sodium bicarbonate and aspartame were weighed at the respective weight ratios shown in Table 3 and mixed using a mortar, and a sensory test of bitterness was carried out using the thus obtained mixed powders. The sensory test was carried out by a panel of two members, by keeping each preparation in the oral cavity for about 20 seconds and then judging the degree of bitterness based on the following evaluation criteria. The results are shown in Table 3.
- A:: Feel no bitterness.
- B:: Feel almost no bitterness.
- C:: Feel bitterness slightly.
- D:: Feel bitterness.
- E:: Feel bitterness strongly.

**Table 3**

| Composition No. | l | m | n | o | p | q |
|---|---|---|---|---|---|---|
| [Component (heat of dissolution cal/g)] | | | | | | |
| Cimetidine | 1 | 1 | 1 | 1 | 1 | 1 |
| Xylitol (-35) | 20 | - | - | - | - | - |
| D-Mannitol (-28.9) | - | 20 | - | - | - | - |
| D-Sorbitol (-24.1) | - | - | 20 | - | - | - |
| Maltitol (-5.5) | - | - | - | 20 | - | - |
| Glucose (-13.8) | - | - | - | - | 20 | - |
| Sucrose (-4.5) | - | - | - | - | - | 20 |
| Sodium bicarbonate | 2 | 2 | 2 | 2 | 2 | 2 |
| Aspartame | 0.02 | 0.02 | 0.02 | 0.02 | 0.02 | 0.02 |

| [Sensory test result] | | | | | | |
|---|---|---|---|---|---|---|
| Panelist A | A | A | A | C | C | D |
| Panelist B | A | A | A | C | C | D |

As is evident from the results shown in Table 3, the bitterness of cimetidine was able to be improved to an undetectable level when sodium bicarbonate was used as the pH adjusting agent, and xylitol, D-mannitol or D-sorbitol having a heat of dissolution of -20 cal/g or less was jointly used as the sugar alcohol (see compositions l to n). However, the bitterness of cimetidine was unable to be improved by a sugar alcohol maltitol not having a heat of dissolution of - 20 cal/g or less (see composition o). Also, the bitterness of cimetidine was unable to be improved by saccharides such as glucose and sucrose (see compositions p and q).

### [Test Example 4] Sensory test of bitterness (4)

Respective powders of cetraxate hydrochloride (pKa: 4.5 (carboxyl group) , pKa: 10.5 (amino group)), ticlopidine hydrochloride (pKa: 6.93), tranexamic acid (pKa: 4.33 (carboxyl group), pKa: 10.65 (amino group)), erythritol, sodium dihydrogen phosphate anhydrous, sodium bicarbonate, aspartame and L-menthol were weighed at the respective weight ratios shown in Table 4 and mixed using a mortar, and a sensory test of bitterness was carried out using the thus obtained mixed powders. The sensory test was carried out by a panel of two members, by keeping each preparation in the oral cavity for about 20 seconds and then judging the degree of bitterness based on the following evaluation criteria. The results are shown in Table 4.
- A:: Feel no bitterness.
- A*:: Feel no bitterness but feel a strong stimulative taste.
- B:: Feel almost no bitterness.
- C:: Feel bitterness slightly.
- D:: Feel bitterness.
- E:: Feel bitterness strongly.
- E*:: Feel strong bitterness and a strong stimulative taste.

**Table 4**

| Composition No. | r | s | t | u | v | w | x | y |
|---|---|---|---|---|---|---|---|---|
| [Component] | | | | | | | | |
| Cetraxate hydrochloride | 1 | 1 | - | - | - | - | 1 | 1 |
| Ticlopidine hydrochloride | - | - | 1 | 1 | - | - | - | - |
| Tranexamic acid | - | - | - | - | 1 | 1 | - | - |
| Erythritol | - | 1 | - | 8 | - | 2 | 1 | 1 |
| Sodium dihydrogen phosphate anhydrous | - | 0.5 | - | 1 | - | 0.5 | - | - |
| Sodium bicarbonate | - | - | - | - | - | - | 0.5 | 0.5 |
| L-Menthol | - | - | - | - | - | - | 0.01 | 0.01 |
| Aspartame | - | 0.01 | - | 0.01 | - | 0.01 | - | 0.01 |

| [Sensory test result] | | | | | | | | |
|---|---|---|---|---|---|---|---|---|
| Panelist A | E | A | E* | A* | D | A | A | A |
| Panelist B | E | A | E* | A* | D | A | A | A |

As is evident from the results shown in Table 4, it was able to improve bitter tastes of cetraxate hydrochloride, ticlopidine hydrochloride and tranexamic acid by jointly using a sugar alcohol and a pH adjusting agent (see compositions s, u and w).

### INDUSTRIAL APPLICABILITY:

According to the invention, it is able to obtain an oral administration preparation in which unpleasant tastes of drugs are improved to such a degree that the unpleasant tastes are completely undetectable, by the joint addition of a sugar alcohol having a heat of dissolution of -20 cal/g or less and a pH adjusting agent. Also, since the amount of the sugar alcohol to be added can be reduced, the pharmaceutical preparation can be miniaturized and has excellent taking ability. In addition, the oral administration preparation of the invention can be produced by a general manufacturing method without requiring a complex production method for example having a number of steps, so that it is economical and has high industrial productivity.

## Claims

1. An oral administration preparation which contains a drug having an unpleasant taste, a sugar alcohol having a heat of dissolution of -20 cal/g or less and a pH adjusting agent.

2. The oral administration preparation according to claim 1, wherein the drug having an unpleasant taste has a basic group in its structure.

3. The oral administration preparation according to claim 1 or 2, wherein the drug having an unpleasant taste is a drug which has a bitter taste.

4. The oral administration preparation according to any one of claims 1 to 3, wherein the drug having an unpleasant taste is an H₂ blocker.

5. The oral administration preparation according to claim 4, wherein the H₂ blocker is a mixture of one or more compounds selected from the group consisting of cimetidine, famotidine, nizatidine and ranitidine hydrochloride.

6. The oral administration preparation according to any one of claims 1 to 3, wherein the drug having an unpleasant taste is a mixture of one or more compounds selected from the group consisting of cimetidine, tranexamic acid and cetraxate hydrochloride.

7. The oral administration preparation according to any one of claims 1 to 6, wherein the sugar alcohol having a heat of dissolution of -20 cal/g or less is a mixture of one or more compounds selected from the group consisting of erythritol, xylitol, mannitol and sorbitol.

8. The oral administration preparation according to any one of claims 1 to 6, wherein the sugar alcohol having a heat of dissolution of -20 cal/g or less is erythritol.

9. The oral administration preparation according to any one of claims 1 to 8, wherein the sugar alcohol having a heat of dissolution of -20 cal/g or less is from 0.1 to 50 parts by weight based on 1 part by weight of the drug having an unpleasant taste.

10. The oral administration preparation according to any one of claims 1 to 8, wherein the sugar alcohol having a heat of dissolution of -20 cal/g or less is from 5 to 10 parts by weight based on 1 part by weight of the drug having an unpleasant taste.

11. The oral administration preparation according to any one of claims 1 to 10, wherein pH value of a 1% (w/v) aqueous solution or 1% (w/v) aqueous suspension of the pH adjusting agent is equal to or higher than the pKa value of the drug having an unpleasant taste or equal to or higher than the pH value of a 1% (w/v) aqueous solution or 1% (w/v) aqueous suspension of the drug.

12. The oral administration preparation according to any one of claims 1 to 11, wherein the pH adjusting agent is a mixture of one or more compounds selected from the group consisting of sodium bicarbonate, sodium dihydrogen phosphate anhydrous and precipitated calcium carbonate.

13. The oral administration preparation according to any one of claims 1 to 12, wherein the pH adjusting agent is from 0.1 to 200 parts by weight based on 1 part by weight of the drug having an unpleasant taste.

14. The oral administration preparation according to any one of claims 1 to 12, wherein the pH adjusting agent is from 0.5 to 7 parts by weight based on 1 part by weight of the drug having an unpleasant taste.

15. An oral administration preparation which contains from 5 to 10 parts by weight of a sugar alcohol having a heat of dissolution of -20 cal/g or less and from 0.5 to 7 parts by weight of a pH adjusting agent, based on 1 part by weight of an H₂ blocker.

16. The oral administration preparation according to any one of claims 1 to 15, wherein it further contains a sweetener and/or a corrective agent.

17. The oral administration preparation according to any one of claims 1 to 15, wherein it further contains aspartame and/or L-menthol.

18. The oral administration preparation according to any one of claims 1 to 17, wherein the dosage forms are tablets, granules, powders, fine subtilaes, solutions or syrups.

19. A method for improving taking ability of an oral administration preparation containing a drug having an unpleasant taste, which is effected by including a sugar alcohol having a heat of dissolution of -20 cal/g or less and a pH adjusting agent.

20. The method for improving taking ability of an oral administration preparation according to claim 19, wherein a sweetener and/or a corrective agent is further included.
